# EUROPEAN PATENT APPLICATION

(11) **EP 4 282 388 A1**
(43) Date of publication of application: **29.11.2023**
(21) Application number: 21920292.6
(22) Date of filing: 22.01.2021
(51) Int. Cl.: A61F 5/56

(54) **SIDE-LYING AUXILIARY BACKPACK ALLOWING TURNING OVER**

(71) Applicant: Wang, Lei, Beijing 100085 (CN)
(72) Inventor: Wang, Lei, Beijing 100085 (CN)
(74) Representative: Bryn Aarflot AS
(86) International application number: PCT/CN2021/073309
(87) International publication number: WO 2022/155894

(57) **Abstract**

A side-lying auxiliary backpack (1) allowing turning over, comprising a left-right symmetrical sheet-like back plate (2), belly bands (7) connected to the back plate (2), optional shoulder bands (5) connected to the back plate (2), optional draw bands (6) connected to the back plate (2) or the belly bands (7), optional leg bands (8) connected to the back plate (2), an openable and lockable main bag (3) located on the back plate (2) and capable of containing a tangible object, and a main airbag (9) of the main bag (3). The backpack (1) is further provided with an auxiliary bag (4) communicated with the main bag (3).

## Description

### TECHNICAL FIELD

The present invention relates to a side-lying auxiliary backpack allowing turning over for assisting people with obstructive sleep apnea and/or snoring during sleep and pregnant women in avoiding a supine sleep posture.

### BACKGROUND

Effects of the sleep posture on obstructive sleep apnea and snoring have long been known. When a patient sleeps on his back, some tissues in a throat of the upper respiratory tract are located above the respiratory tract and in a relaxed state, and collapse into the respiratory tract under the effect of gravity, thereby resulting in an increase in respiratory resistance. If the above tissue is hypertrophic or hyperplastic, obstruction of the respiratory tract will be aggravated. When a patient sleeps on one side, the above tissues point to one side of the body under the effect of gravity, the respiratory tract will not be pressed, thereby reducing the chance of breathing obstruction. Studies have shown that sleeping on the back causes at least twice as much sleep apnea and snoring as sleeping on the side. For many people, avoiding the supine sleep posture can reduce or even prevent sleep apnea and/or snoring.

The sleeping posture of pregnant women is closely related to the health of mother and child. The uterus gradually enlarges in the late pregnancy. When lying on the back, the uterus may compress the kidney, which may reduce the renal blood flow and decrease the glomerular filtration rate, which will affect the health of pregnant women and fetuses. In addition, when pregnant women lie on the back, the enlarged uterus may compress the inferior vena cava, which may hinder the blood reflux of the inferior vena cava and cause edema and varicose veins of the lower limbs. Meanwhile, due to a decrease of returned blood volume, blood supply of the whole body decreases, causing chest tightness, nausea, vomiting, blood pressure drop and other symptoms, which are medically called "supine hypotension syndrome". It is generally emphasized that it is not appropriate to lie on the back for a long time after 6 months of pregnancy.

In terms of avoiding supine sleep, the prior art provides only limited options and is highly limited. Some patients sew tennis balls, golf balls and other objects on the back of pajamas to form bulges, hoping that the pressure of the bulges on the back will help them break away from the supine posture. The limitation of the technique is as follows: firstly, the bulge is easy to move along with clothes and deviate from the middle position of the back; secondly, the users cannot adjust their side-lying angles; thirdly, the bulge is small in size and is difficult to achieve the necessary side-lying angle, especially when the mattress is soft, the bulge may fall into the bed and cause failure; and fourthly, local pressure on bulges may cause injuries to the back or spine.

US20120167895A1 describes a band capable of avoiding supine sleep, and is similar to WO2007130931A1 and WO2016176632A1. Such techniques include a band around the human torso and a bulge fixed to the band and arranged in the middle position of the back, and such a bulge is used to destroy the stable balanced state of supine lying. The limitation of this technique is as follows: firstly, if the bulge is small in size, it is difficult to achieve the necessary side-lying angle, especially when the mattress is soft, the bulge may fall into the bed and cause failure, and if the bulge is large in size, it is difficult for the users to turn over; secondly, the user cannot adjust the side-lying angle; thirdly, because the band is fixed around the torso and easily rotates around the torso, the bulge deviates from the middle position of the back; and fourthly, the binding force is concentrated on one band, which is easy to cause discomfort, especially if the band is wrapped around the chest, it will cause discomfort to female users.

EP3117805A1 discloses a wearable side-lying auxiliary device. In the device, a waist pillow is fixed on the lower back of a waistcoat, and the waist pillow is used to prevent the user from lying on the side to lying on the back. The limitations are as follows: firstly, if the size of the waist pillow is large, it is difficult for the user to turn over, and if the size of the waist pillow is small, the necessary side-lying angle cannot be achieved; secondly, the user cannot adjust the side-lying angle; thirdly, it is impossible to give attention to firmness and comfort, if the waistcoat is thick, it is not comfortable enough; if the waistcoat is thin, the waist pillow is easy to move with the waistcoat; and fourthly, the waistcoat has poor adaptability to different statures.

A backpack capable of avoiding supine lying disclosed in DE 202014003665U1 is an advanced technical solution now. The backpack consists of a bag located in the middle of the back and a shoulder band and a belly band for fixation. Through fixation at three points including the waist and two shoulders, the backpack can be prevented from rotating along the torso, and the binding force can also be dispersed well. Through fillers, the backpack can ensure necessary size and strength to completely avoid supine lying and provide a better side-lying angle. The limitations are as follows: firstly, if the backpack is large in size, it is difficult for the user to turn over; if the backpack is small in size, the necessary side-lying angle cannot be achieved; secondly, since the size of the backpack is not adjustable, the user cannot adjust the side-lying angle, if the side-lying angle is changed by adjusting the size of the filler, the backpack may not be matched with the filler; thirdly, only fixation at double shoulders is applied, and the applicability is insufficient, most users will lean on the backpack when lying on their sides, and the shoulder band on the lower side of the body will be forced to tighten the lower shoulder, and cause discomfort; and fourthly, an elastic band and an adjustment band are adopted to improve size adaptability, and the structure is complex and the adjustment range is small.

### SUMMARY OF THE INVENTION

The present invention provides a turning-over backpack configured to prevent people with obstructive sleep apnea and/or snoring and pregnant women from a supine sleep posture. In the present invention, through an auxiliary bag structure, the height of the backpack can be converted into width or/and length, so that the user can turn over during sleep to ensure comfort of sleep. Adjusting the size of the auxiliary bag can indirectly adjust the minimum bulged height of the backpack to adapt to different users. The size of the main bag of the backpack can be adjusted, on the one hand, lying on the back will be completely avoided, and on the other hand, users can be helped to lie on their sides at different angles. The present invention also provides a plurality of reliable fixing modes, among which the user can choose the firmest and most comfortable one. In addition, the shoulder band, the draw band, the belly band, the back plate and other parts use materials that can be bonded with a hook and loop, and in combination with the folding and pasting technology, the adaptability to different statures is improved. To sum up, the present invention avoids disadvantages of the prior art to a great extent, and provides a side-lying auxiliary technology with more practicability and comfort.

To achieve the above object, the present invention provides a side-lying auxiliary backpack allowing turning over, including:
1. a back plate, wherein the back plate is a left-right symmetrical sheet-like object. Preferably, the back plate is in left-right symmetry with the spine as a symmetry axis when the back plate is worn. The back plate is connected to a belly band for fixing the waist and belly. When the belly band is worn, the belly band surrounds the torso between the chest and the belly. Optionally, the back plate is connected to shoulder bands, usually two shoulder bands, and the shoulder bands are configured to fix the backpack on the upper part of the torso. Optionally, the back plate is connected to leg bands, usually one or two leg bands, and the leg bands are configured to fix the backpack on the hips and/or legs. Optionally, the back plate or belly band is also connected to draw bands, usually two to four draw bands, and the draw bands are connected to and used in combination with the shoulder bands or/and leg bands to realize fixation of the backpack on the torso. In a particular embodiment, the back plate can be reduced and fused into a part of the main bag described below.
2. One or more openable and lockable and lockable bags (hereinafter referred to as "main bags") on the back plate. Preferably, when the main bag is worn, the main bag is located on the back or/and in the middle of hips perpendicular to the spine direction. The preferred material of the main bag is a non-elastic material, to avoid rotation along the torso during use. The main bag may contain one or more tangible objects as fillers. The whole or part of the filler may be an elastomer. Preferably, the filler is an inflatable and deflatable airbag (hereinafter referred to as "main airbag"). Generally, the overall size and shape of the filler in the main bag are substantially the same as the size and shape of the main bag, to avoid the filler from being unable to be incorporated into the main bag or from displacing in the main bag. After the filler is added, the main bag bulged on the back may prevent the user from lying on his side to lying on his back. By adjusting the size and/or shape of the main bag and the filler, the user can be provided with an ideal side-lying angle. In order to more conveniently adjust the side-lying angle, an adjustment element can be arranged on the main bag to adjust its size and/or shape and synchronously adjust the filler for matching. When the filler is a preferred main airbag, the change of the main airbag can be matched by inflating and deflating.
3. One or more bags (hereinafter referred to as "auxiliary bags") in communication with the main bag. Preferably, the auxiliary bag is located close to the back plate. In a side lying state, the filler will not fill the auxiliary bag. When the user turns over from lying on the side to lying on the back, the pressure on the filler increases and elastic deformation occurs, and part of the filler begins to fill the auxiliary bag. At this time, the height of the filler is gradually converted into width and/or length, causing the bulged height of the main bag to decrease and become flat. When the user lies on his back, the pressure on the filler is the maximum, and the auxiliary bag is filled. The main bag is at the minimum bulged height, so that the user can overcome the obstruction of the backpack and turn to the other side. After the user reaches the other side, the pressure on the filler decreases and gradually recovers to its original shape, and the angle of side lying gradually increases, and an ideal angle and a stable balanced state is reached.

In order to adapt to different users, adjustment elements can be set on the auxiliary bag to adjust the size of the auxiliary bag, so as to control the minimum bulged height of the main bag under pressure. On the one hand, the user can overcome this obstacle to complete turning over; and on the other hand, the user cannot reach a stable balanced state in the supine state during the turning-over process, and the user must continue to turn over to the side lying state.

As an improvement, when the filler is the main airbag, the main airbag is communicated with an elastic airbag (hereinafter referred to as "auxiliary airbag") at the communicating part between the main bag and the auxiliary bag. When the user lies on the side, the auxiliary airbag is in a contracted state. When the user turns over, the main airbag is pressurized to enable gas to enter the auxiliary airbag, and the auxiliary airbag is inflated in the auxiliary bag, so that the main bag and the main airbag become flat. The main bag is flattest when the auxiliary airbag is filled with auxiliary bags. As a further improvement, a connecting valve may be arranged at the communicating part between the main airbag and the auxiliary airbag, and the auxiliary airbag can be detachably replaced at the connecting valve to adapt to different usage requirements.

As a further improvement, when the main bag is located at the waist and the hip, the connecting part between the main bag and the auxiliary bag and the connecting part between the main airbag and the auxiliary airbag can be bent to the front side of the body through the crotch from back to front, such that the auxiliary bag and the auxiliary airbag are located on the front side of the body. When the main bag is pressurized, gas in the main airbag enters the auxiliary airbag, and the auxiliary airbag expands on the front side of the human body. Its advantage is that the auxiliary airbag is not compressed during the turning-over process, the expansion range is large, and the bulged height of the main bag can be adjusted in a large range.

4. The two shoulder bands are connected to the back plate by sewing or connecting elements, and a length adjustment device can be installed on the shoulder bands. When two shoulder bands wrap around the two shoulders respectively, fixation at double shoulders can be realized. When two shoulder bands are crossed in front of the chest (each shoulder band goes around the chest through the shoulder on one side of the body and reaches the waist and belly on the other side), cross fixation at the chest can be realized. As an improvement, two draw bands connected to the back plate or the belly band can be arranged, and the draw bands can be connected to the shoulder band through a connecting element. When the shoulder bands are respectively connected to the draw bands on opposite sides of the body at the front side of the body, cross fixation at the chest can be realized. When the shoulder bands are respectively connected to the draw bands on the same side of the body at the front side of the body, fixation at double shoulders can be realized. As a further improvement, each of the two front sections of the shoulder bands has a loop. The front section of the two draw band is attached with a first hook and loop, and the middle section is attached with a second hook and loop, and the overall draw band is made of a material that can be bonded with a hook and loop. When the two draw bands pass through the loops of the shoulder bands on the opposite side of the body on the front side of the body, the draw bands are folded back and glued to themselves with the first hook and loop, to realize cross fixation at the chest. When the two draw bands pass through the loops of the shoulder bands on the same side of the body and are folded back, the draw bands are glued to themselves with the second hook and loop, to realize fixation at double shoulders. The above design provides two reliable fixing modes for users to choose. The connecting point between the draw band and the shoulder band can be conveniently set on the front side of the body, which is convenient for fastening and adjustment. The hook and loop of the draw band can be attached to any position of the draw band between the shoulder and the waist, and its length can be adjusted to cover users of all statures, such adjustment devices as elastic bands, extension bands, and adjustment clips do not need to be additionally set. Finally, the loop of the shoulder band can better lock the position of the shoulder band and the draw band, and the bonding with the hook and loop is more secure after the shoulder band is folded back.

5. One end of the leg band is connected to the back plate by sewing or a connecting element, and a length adjustment device can be arranged on the leg band. When the backpack is provided with a leg band, the other end of the leg band goes around to the front side of the body through the crotch from back to front, and is connected to the belly band. When the backpack is provided with two leg bands, the other end of the leg band passes through the crotch from back to front, and then goes around the root of the thigh to be connected to the back plate or the belly band. When two leg bands are set, if the other end of each leg band is connected to the back plate or belly band on the opposite side of the body, cross fixation at the legs can be achieved; if the leg bands are connected to the back plate or belly band on the same side of the body, fixation around the legs can be achieved. As an improvement, a draw band connected to the back plate or the belly band can be arranged, and the draw band can be connected to the leg band through a connecting element. In the case of setting two leg bands and two draw bands, when the leg bands pass through the crotch from back to front, the leg bands are respectively connected to the draw bands on the opposite side of the body, such that cross fixation at the legs can be achieved; if the leg bands are respectively connected to the draw band on the same side of the body, fixation around the legs can be achieved.

6. The belly band is connected to the back plate by sewing or connecting elements. As an improvement, two belly bands can be arranged, and the belly bands as a whole are made of materials that can be bonded with a hook and loop. One end of each belly band is connected to the back plate, and the other end is connected to the front of the body by a hook and loop. As a further improvement, the front section of one belly band has a loop, and the other belly band passes through from bottom to top, and is glued to its surface with a hook and loop after being folded back.

### BRIEF DESCRIPTION OF THE DRAWINGS

Fig. 1 is a front view of a backpack in the first embodiment of the present invention.
Fig. 2 is a rear view of a backpack in the first embodiment of the present invention.
Fig. 3 is a perspective view of a side of a backpack according to the first embodiment of the present invention.
Fig. 4 is a schematic diagram of a backpack assisting in side lying according to the first embodiment of the present invention.
Fig. 5 is a schematic diagram of a fixing mode of a backpack according to the first embodiment of the present invention.
Fig. 6 shows the change of the main airbag of the backpack of the first embodiment of the present invention during a turning-over process.
Fig. 7 is a schematic diagram showing the backpack in different turning-over states according to the first embodiment of the present invention.
Fig. 8 shows the changes of the main airbag and the auxiliary airbag of the backpack according to the second embodiment of the present invention during the turning-over process.
Fig. 9 is a schematic diagram of a connecting valve of the main airbag and the auxiliary airbag of the backpack according to the second embodiment of the present invention.
Fig. 10 shows the change of the main airbag of the backpack according to the third embodiment of the present invention during the turning-over process.
Fig. 11 is a front view of a backpack according to the fourth embodiment of the present invention
Fig. 12 is a schematic diagram of a backpack fixing mode according to the fourth embodiment of the present invention.

### DETAILED DESCRIPTION

The present invention is further described with reference to the accompanying drawings.

The backpack of the present invention can be easily manufactured from known materials without great technical effort.

The term "backpack" as used in the present invention refers to one band or a set of closed bands which can be fixed on the torso of the body, and the band is provided with one or more bags with the main body located on a back side of the human body and being capable of accommodating tangible objects.

The term "back plate" used in the present invention refers to a left-right symmetrical sheet-like object, and is located on the back or/and hip when the backpack is worn.

The terms "primary bag" and "auxiliary bag" used in the present invention both refer to a bag capable of accommodating tangible objects, in addition to the case in which the bag itself is capable of accommodating tangible objects, the case in which the bags together with the back plate of the present invention constitute a bladder-shaped structure to accommodate tangible objects is also included.

The term "draw band" as used in the present invention refers to a band or a set of bands with one end of the band being connected to the back plate or the belly band and the other end being connected to the shoulder band or the leg band for fixing a backpack on the human torso.

The term "hook and loop" used in the present invention is intended to mean two bands used in pairs, one band has a hook structure on the surface and the other band has an annular structure on the surface, and the two bands can be bonded (hooked) together face to face. In this text, owing to technological advances, backpacks can use special surface materials, and these materials can replace one piece of band in the traditional "hook and loop" to be bonded (hooked) with another piece of band. Therefore, in this text, the "hook and loop" refers to both a traditional pair of bands and a single band that can be adhered to a backpack.

The term "side-lying angle" used in the present invention refers to an included angle formed between the back of the person lying on his side and the bed surface, and the included angle ranges from 0 degree to 180 degrees. The closer the sleeping posture is to lying on the back, the closer the side-lying angle is to 0 degree; the closer the sleeping posture is to lying on the stomach, the closer the side-lying angle is to 180 degrees. An ideal side-lying angle is around 90 degrees.

Embodiments of the present invention are shown in the drawings and described in details below.

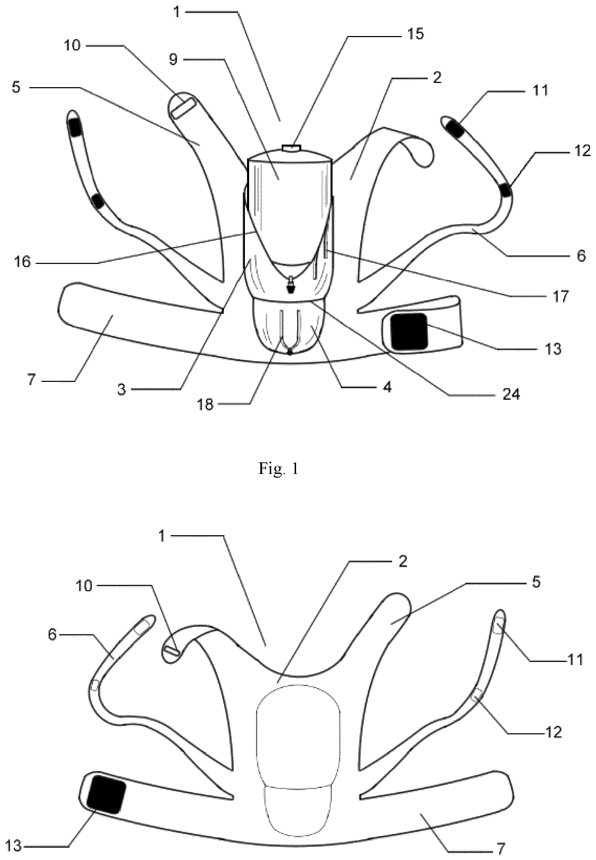

Figs. 1, 2, 3, 4, 5, 6, 7 are first embodiment of the present invention. The backpack 1 includes a back plate 2, and the back plate 2 is a sheet-shaped object, and is padded on the back in left-right symmetry with the spine serving as a center line. The effect of the back plate 2 is to connect the shoulder band 5, the draw band 6 and the belly band 7, to fix the main bag 3 to avoid displacement and rotation simultaneously, and to disperse the supporting force of the main bag 3 and the main airbag 9 to the back. The back plate 2 can be of a multi-layer structure, the side close to the back can be made of a skin-friendly material, and the interlayer can contain elastic cushioning materials to enhance comfort, and can also contain harder and tougher plate-shaped shaping materials to avoid deformation of the backpack 1.

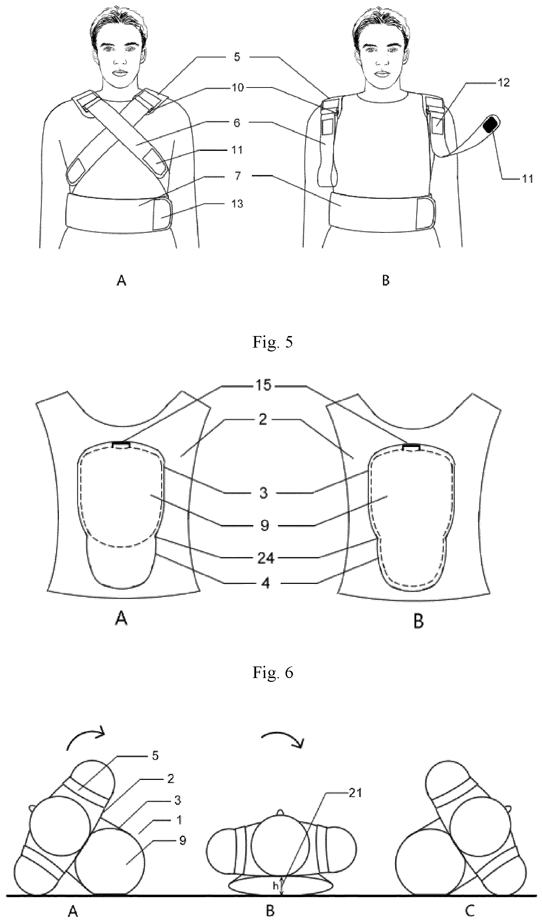

Two shoulder bands 5 are respectively connected to both sides of the upper end of the back plate 2, and a loop 10 is arranged at the front section of the shoulder band. Two draw bands 6 are respectively connected to both sides of the middle and lower part of the back plate 2. The front section of the draw band is provided with a first hook and loop 11, and the middle section is provided with a second hook and loop 12. The entire draw band 6 is made of materials that can be bonded with a hook and loop. When the backpack is worn, one draw band 6 is folded back after passing through the loop of a shoulder band 5, and glued to the surface of the draw band 6 with the hook and loop 11 or/and 12, to realize fixation of the backpack 1 on the upper part of the torso. Wherein, the first hook and loop 11 is used for cross fixation at the chest (Fig. 5-A), and the second hook and loop 12 is used for fixation at double shoulders (Fig. 5-B). The specific method is described in details in Fig. 5.

Both sides of the bottom of the back plate 2 are also connected to two belly bands 7 respectively, the belly bands 7 are provided with a hook and loop 13, and the entire belly band 7 is made of materials that can be bonded with a hook and loop. When the backpack is worn, the hook and loop 13 is bonded to the surface of another belly band 7, so that the two belly bands 7 are connected on the front side of the body.

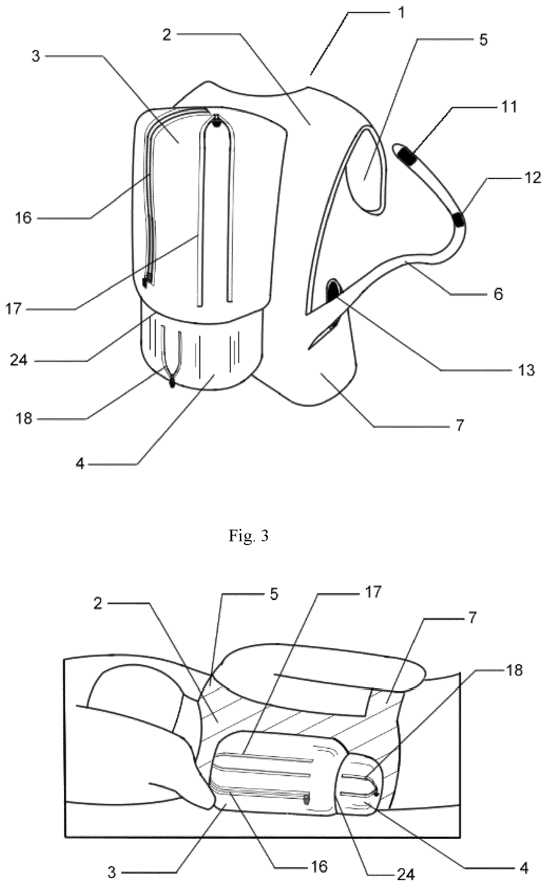

The middle part of the back plate 2 is provided with an openable and lockable main bag 3 (the main bag 3 is in an open state in Fig. 1 and is in a closed state in Fig. 3), and the opening, closing and locking device on the main bag 3 is a zipper 16 in the direction of the head and feet. The filler of the main bag 3 can be put in here. The filler of the main bag 3 is a main airbag 9. The main airbag 9 is provided with an air nozzle 15, and the air nozzle 15 can close and open the main airbag 9 to realize inflation and deflation. The size and shape of the main airbag 9 after inflation are substantially the same as the size and shape of the main bag 3. The airbag is used as a filler for easy portability, and the side-lying angle can be adjusted by inflation and deflation, and the advantage of easy deformation can also be used to achieve the turning-over action.

In the present embodiment, the side-lying angle can be adjusted by controlling the fullness of the main airbag 9. Simultaneously, an adjustment zipper 17 is arranged on the main bag 3, such that the main bag 3 can be matched with the main airbags 9 of different degrees of fullness. The left and right halves of the adjustment zipper 17 are fixed on the outer surface of the main bag 3 and keep a certain distance. By opening and closing the zipper 17, the surface area of the main bag 3 can be adjusted, and then the size and/or shape of the main bag 3 can be adjusted.

An auxiliary bag 4 communicated with the main bag 3 is arranged below the main bag 3, and a connecting part 24 is arranged at the communicating part between the main bag 3 and the auxiliary bag 4. The auxiliary bag 4 can provide a turning-over function for the backpack 1, and the specific process is described in details in Fig. 6 and Fig. 7. The auxiliary bag 4 is provided with an adjustment zipper 18 to adjust its size. The left and right halves of the adjustment zipper 18 are fixed on the outer surface of the auxiliary bag 4 and keep a certain distance. By opening and closing the zipper 18, the surface area of the auxiliary bag 4 can be adjusted, and then the size of the auxiliary bag 4 can be adjusted, thereby indirectly regulating the minimum bulged height 21 of the main airbag 9 (see Fig. 7).

Fig. 4 shows the situation that the backpack 1 assists in lying on the side in the first embodiment. The shoulder band 5, the draw band 6 and the belly band 7 fix the backpack 1 on the back of the user. Under the condition of fixing at double shoulders and the waist, the backpack 1 is very firm and cannot rotate around the torso. Simultaneously, the shoulder band 5, the draw band 6 and the belly band 7 disperse the binding force brought by fixing the backpack 1. The main airbag 9 props up the main bag 3, such that the backpack 1 bulges on the back of the user, supports the back of the user, assists the user in keeping lying on the side, and hinders the user from turning to lie on the back simultaneously. By regulating the fullness of the main airbag 9 and adjusting the adjustment zipper 17 of the main bag 3, the size of the backpack 1 can be adjusted, thereby adjusting the side-lying angle.

Fig. 5 shows two ways of fixing the backpack 1 in the first embodiment. The draw band 6 and the belly band 7 are integrally made of materials that can be bonded with a hook and loop. Fig. 5-A shows cross fixation at the chest of the backpack 1. At this time, two draw bands 6 pass through the loop 10 of the front section of the shoulder band 5 on a different side of the body from bottom to top respectively, and are glued on the surface of the draw band 6 with the first hook and loop 11 after being folded back. Since the draw band 6 consumed for cross fixation at the chest is longer, the position of the first hook and loop 11 is just matched with the draw band 6. At this moment, the second hook and loop 12 has not yet passed through the loop 10 or has just passed through the loop 10, and can be glued on the surface of the draw band 6 to assist in fixation. It can be seen from Fig. 5-A that the variable bonding position of the hook and loop 11 is between the loop 10 and the connecting part between the draw band 6 and the back plate 2. If a shorter shoulder band 5 is used, preferably 15cm, the adjustable range of the draw band 6 can be adapted to users of all statures.

Fig. 5-B shows fixation at double shoulders of the backpack 1. At this time, the two draw bands 6 pass through the loop 10 of the front section of the shoulder band 5 on the same side of the body from bottom to top, and are glued to the surface of the draw band 6 with the second hook and loop 12 after being folded back. Since the draw band 6 consumed in fixation at double shoulders is relatively short, the position of the second hook and loop 12 is just matched with the draw band 6. The redundant part of the front section of the draw band 6 can be fixed on the shoulder band 5, the draw band 6 or the belly band 7 with the first hook and loop 11, to avoid being scattered. The variable bonding position of the hook and loop 12 is between the loop 10 and the connecting part between the draw band 6 and the back plate 2. If a shorter shoulder band 5 is used, preferably 15cm, the adjustable range of the draw band 6 is suitable for users of all statures.

The two belly bands 7 in the present embodiment are made of a material that can be bonded with a hook and loop, and one of the belly bands 7 is provided with a hook and loop 13. When the backpack is worn, the hook and loop 13 is bonded to the surface of another belly band 7, such that the two belly bands 7 are connected on the front side of the body, so that fixation at the waist and belly of the backpack 1 is realized.

Fig. 6 and Fig. 7 have introduced the turning-over function of the backpack 1 in the present embodiment. In Fig. 6-A and Fig. 7-A, the user keeps lying on his side and has not turned over yet, and the main airbag 9 does not enter the auxiliary bag 4. Next, the user starts to turn over, and the pressure on the main airbag 9 increases, and its elastic outer wall expands and gradually enters the auxiliary bag 4, such that the bulged height of the main bag 3 and the main airbag 9 is reduced. In Fig. 6-B and Fig. 7-B, the user turns to the supine state, the main airbag 9 is filled with the auxiliary bag 4, and the main bag 3 and the main airbag 9 are compressed to the minimum bulged height 21 at this time. However, the user cannot reach a stable balanced state in the supine state, and continues to turn over to the other side according to his willingness to turn over, and begins to enter the side lying state. In Fig. 7-C, the user returns to the side lying state on the other side, the pressure on the main airbag 9 decreases and elastically shrinks, and the side-lying angle increases, thereby achieving an ideal side-lying angle and a stable balanced state.

In the present embodiment, the entire surface of the main airbag 9 can be made of elastic materials, or only the auxiliary bag 4 can be made of elastic materials. By selecting a suitable elastic material, the pressure threshold at which the main airbag 9 deforms can be adjusted to adapt to different users.

In the present embodiment, adjusting the size of the auxiliary bag 4 can regulate the minimum bulged height 21 of the main bag 3 and the main airbag 9 under pressure, so as to adapt to different users. On the one hand, the user can overcome this obstacle to turn over, and on the other hand, the user cannot achieve a stable balanced state in the supine state, and must turn to the side lying state.

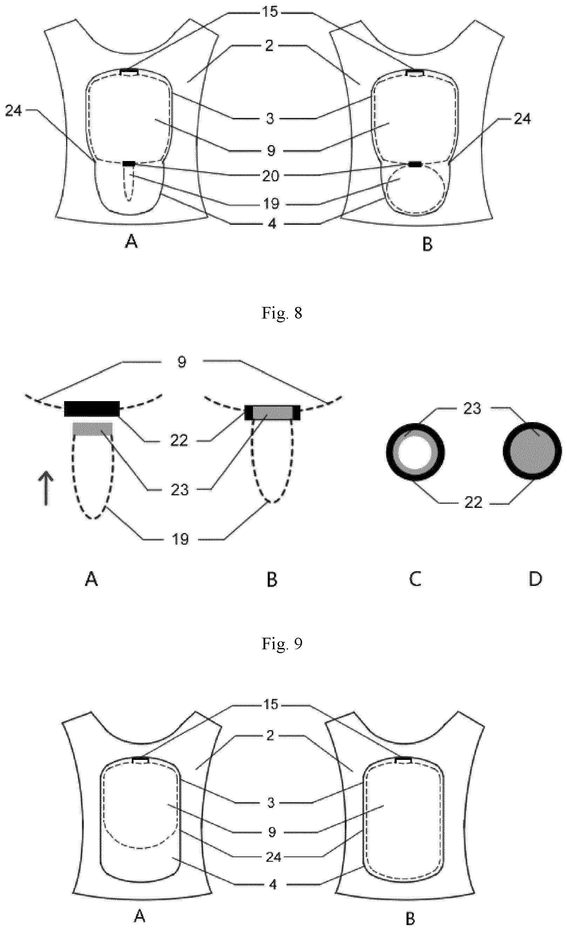

Fig. 8 and Fig. 9 are the second embodiment of the present invention, and its overall structure is similar to that in the first embodiment. The special part of the present embodiment lies in that the main airbag 9 is at the communicating part between the main bag 3 and the auxiliary bag 4, and its outer wall communicates with an elastic auxiliary airbag 19 through a connecting valve 20. In Fig. 8-A, the user keeps lying on his side, and the auxiliary airbag 19 is in a contracted state at this moment. In Fig. 8-B, the user turns to the supine state in the turning-over process. At this time, the main airbag 9 is pressurized such that the gas enters the auxiliary airbag 19, and the auxiliary airbag 19 expands and fills the auxiliary bag 4, such that the main bag 3 and the main airbag 9 become flat. By selecting a suitable elastic material, the pressure threshold at which the auxiliary airbag 19 deforms can be adjusted to adapt to different users.

In the present embodiment, the connecting valve 20 is composed of a valve frame 22 and a valve core 23. The valve frame 22 is fixed on the main airbag 9, and the valve core 23 is fixed on the auxiliary airbag 19. The valve frame 22 and the valve core 23 can be sealed and connected by threads, rotating buckles, plugging and the like. The mutual nesting and insertion relationship of the valve frame 22 and the valve core 23 can be interchanged in other embodiments. Via the connecting valve 20, auxiliary airbags 19 of different elasticity can be used instead. Fig. 9-A is a side view before the valve frame 22 is connected to the valve core 23. Fig. 9-B is a side view after the valve frame 22 is connected to the valve core 23. Fig. 9-C is a top view after the valve frame 22 is connected to the valve core 23. As shown in Fig. 9-D, if the auxiliary airbag 19 is not required, the valve core 23 can be changed into a cover to completely seal the connecting valve 20. In addition, with the development of new requirements, an airflow control device or other smart devices may be arranged inside the valve core 23 as considered subsequently, in order to improve the function of the backpack 1.

Fig. 10 shows a third embodiment of the present invention, and its overall structure is similar to that of the first embodiment. A special part of the present embodiment lies in that the cross sections of the main bag 3 and the auxiliary bag 4 and the connecting part 24 are substantially identical, at this time, the main bag 3 and the auxiliary bag 4 have been integrated as a whole. In Fig. 10-A, the user keeps lying on his side, at this time, the main airbag 9 occupies the space of the main bag 3 and does not enter the area of the auxiliary bag 4. In Fig. 10-B, when the user turns to the supine state during the turning-over process, the main airbag 9 is pressed and elastically expands to fill the space of the auxiliary bag 4, while the main bag 3 and the main airbag 9 become flat, the user can complete the turning-over process. In the present embodiment, the main airbag 9 may be possibly displaced in the direction of the head and feet in the space in which the main bag 3 and the auxiliary bag 4 are fused in the side lying state, but as long as it is controlled within a reasonable range, the side lying posture of the user is not affected, because the main airbag 9 does not displace in a direction of the back vertical to the spine, the turning-over function is also not affected, because in the side lying state, in the space in which the main bag 3 is fused with the auxiliary bag 4, the space which is not filled by the main airbag is not changed.

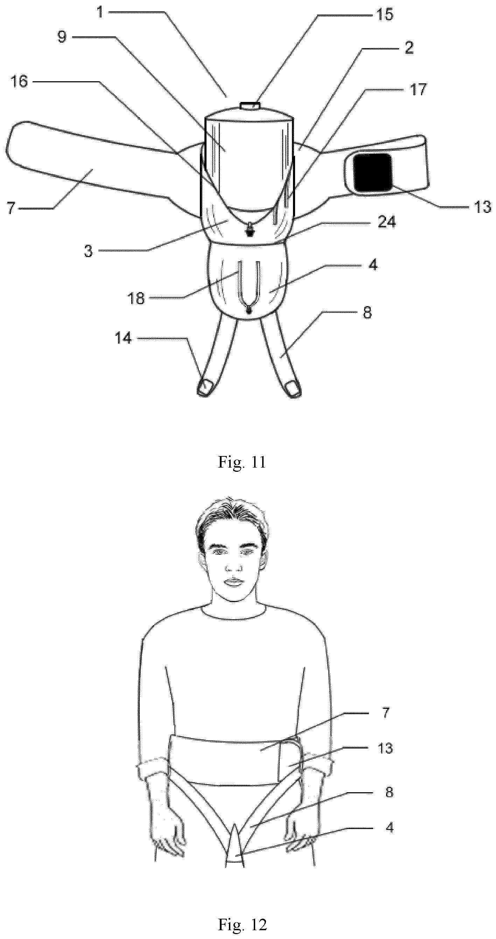

Figs. 11 and 12 show a fourth embodiment of the present invention. The structures of the main airbag 3, the auxiliary airbag 4, the main airbag 9 and its accessory device of the present embodiment are similar to the structures in the first embodiment. A special part of the present embodiment lies in that, when the backpack is worn, the main bag 3 is located at the waist and hip, and the auxiliary bag 4 is located at the hip and the rear side of the thighs. The shape and size of the back plate 2 are adjusted accordingly. In terms of the fixing mode, the shoulder band 5 and the draw band 6 are not used in the present embodiment, but a mode in which the belly band 7 and the leg band 8 are matched is adopted. When the backpack is worn, the fixing mode of the belly band 7 is the same as the fixing mode in the first embodiment. One end of the leg band 8 is fixed at the lower part of the back plate 2, and the other end passes through the crotch of the user from back to front, then passes through the thigh, and is finally connected to the outer surface of the belly band 7 on the side of the body by a hook and loop 14, to realize fixation around the legs. In the present embodiment, fixation at three points including two legs and the waist is adopted, which may prevent the backpack 1 from rotating around the torso, and may also effectively disperse the binding force. In addition, the auxiliary bag 4 is arranged below the hip, which is more conducive to turning over. Since in the turning-over process, double legs are usually slightly bent during the turning-over process, at this time, the pressure of the thighs on the auxiliary bag 4 is less, and the auxiliary bag 4 is more inflated than that in the first embodiment when the user turns to the supine state, thereby more effectively reducing the minimum bulged height of the main bag 3 and the main airbag 9. The present embodiment has the advantages of easy turning over, comfortable wearing, convenient disassembly and the like, and is especially suitable for pregnant women.

Finally, it should be noted that the above embodiments are merely intended to illustrate rather than limiting the technical solution of the present invention. While the present invention has been described in details with reference to the embodiments, those skilled in the art should understand that modifications or equivalent substitutions made to the technical solution of the present invention do not depart from the spirit and scope of the technical solution of the present invention and should fall within the scope of the claims of the present invention.

## Claims

1. A side-lying auxiliary backpack allowing turning over, comprising: a left-right symmetrical sheet-like back plate, belly bands connected to the back plate, optional shoulder bands connected to the back plate, optional draw bands connected to the back plate or the belly bands, optional leg bands connected to the back plate, an openable and lockable and lockable main bag located on the back plate and capable of containing a tangible object, and tangible fillers of the main bag, wherein the backpack is further provided with an auxiliary bag communicated with the main bag.

2. The backpack of claim 1, wherein the back plate is fused as a part of the main bag.

3. The backpack of claim 1, wherein the main bag is provided with an adjustment element to adjust its size and/or shape.

4. The backpack of claim 1, wherein the auxiliary bag is provided with an adjustment element to adjust its size and/or shape.

5. The backpack of claim 1, wherein the main bag filler is an inflatable and deflatable main airbag.

6. The backpack of claim 5, wherein the main airbag is communicated with an auxiliary airbag.

7. The backpack of claim 6, wherein a connecting valve is arranged at a communicating part between the main airbag and the auxiliary airbag.

8. The backpack of claim 6, wherein the auxiliary bag and the auxiliary airbag are placed on a front side of the body through a crotch.

9. The backpack of claim 1, wherein the shoulder band can realize two fixing modes: cross fixation at the chest and fixation at double shoulders.

10. The backpack of claim 1, wherein the shoulder band and the draw band can be connected to realize two fixing modes: cross fixation at the chest and fixation at double shoulders.

11. The backpack of claim 10, wherein the front section of the shoulder band is provided with a loop, a front section of the draw band is provided with a first hook and loop, and a middle section is provided with a second hook and loop.

12. The backpack of claim 1, wherein the leg band can realize two fixing modes:
cross fixation at legs and fixation around the legs.

13. The backpack of claim 1, wherein the leg band and the draw band can be connected to realize two fixing modes: cross fixation at legs and fixation around the legs.
